# EUROPÄISCHE PATENTSCHRIFT

(11) **EP 0 460 412 B1**
(45) Veröffentlichungstag und Bekanntmachung des Hinweises auf die Patenterteilung: **15.03.1995**
(21) Anmeldenummer: 91107293.2
(22) Anmeldetag: 06.05.1991
(51) Int. Cl.: C07D 233/88

(54) **Verfahren zur Herstellung von Allantoin**
Process for the preparation of allantoin
Procédé pour la préparation d'allantoine

(30) Priorität: 05.06.1990 AT 1210/90
(43) Veröffentlichungstag der Anmeldung: 11.12.1991
(73) Patentinhaber: Chemie Linz GmbH, A-4021 Linz (AT)
(72) Erfinder: Schermanz, Karl, Mag. Dr., A-8010 Graz (AT); Fitzinger, Klaus, A-4020 Linz (AT)
(74) Vertreter: Kunz, Ekkehard, Dr.

(56) Entgegenhaltungen:
- EP-A- 0 099 981
- DE-A- 1 939 924
- DE-A- 2 714 938
- DE-A- 2 717 698
- CHEMICAL ABSTRACTS, vol. 97, no. 11, 13 September 1982 Columbus, Ohio, USA, Seite 771, ref. no. 92275x ; & JP-A-5750971
- CHEMICAL ABSTRACTS, vol. 97, no. 7, 16 August 1982 Columbus, Ohio, USA Seite651; ref. no. 55807B; & JP-A-57-50970

## Beschreibung

Die Erfindung betrifft ein Verfahren zur Herstellung von Allantoin.

Allantoin (2,5-Dioxo-4-imidazolinyl)harnstoff und dessen Salze und Metallkomplexe stellen pharmazeutisch wirksame Verbindungen dar, die in der Krebstherapie und -prophylaxe Verwendung finden.

Aus DE-A 19 39 924 ist ein Verfahren zur Herstellung von Allantoin in wäßrigem Medium bekannt, bei dem Glyoxylsäure und Harnstoff in Gegenwart einer Mineralsäure oder einer organischen Sulfonsäure umgesetzt werden, wobei Allantoin in Ausbeuten von weniger als 60 % erhalten wird.

In Chemical Abstracts, Vol 97, 55807 ist ein Verfahren zur Herstellung von Allantoin durch Umsetzung von Glyoxylsäureestern mit Harnstoff in Gegenwart eines Kondensationsmittel im sauren oder basischen Milieu beschrieben. Pro Äquivalent Glyoxylsäureester werden 0,5 - 4,0 Äquivalente Kondensationsmittel eingesetzt. Ferner ist aus Chemical Abstracts, Vol. 97,92275 ein Verfahren zur Herstellung von Allantoin durch Umsetzung eines Essigsäurederivats mit Harnstoff in Gegenwart eines Kondensationsmittels in saurem oder basischem Milieu bekannt. Pro Äquivalent Essigsäurederivat werden 0,1 bis 4,0, vorzugsweise jedoch 0,5 - 4,0 Äquialente Kondensationsmittel eingesetzt.

Beide Verfahren liefern in einer einstufigen Reaktion, die entweder im sauren oder im basischen Milieu durchgefüfhrt wird, Allantoin in einer Ausbeute von 67 - 76 %. In beiden Verfahren wird eine große Menge an Kondensationsmittel verwendet, das nach der Isolierung des Allantoins im Abwasser verbleibt und neutralisiert werden muß. Eine großtechnische Anwendung dieser Verfahren ist daher nur schwer möglich.

Es konnte nun unerwarteterweise ein großtechnisch anwendbares Verfahren zur Herstellung von Allantoin gefunden werden, bei dem ausgehend von Glyoxylsäuremethylestermethylhemiacetal unter Verwendung katalytischer Mengen an anorganischer Säure Allantoin in einer zuerst sauren dann basischen Reaktion mit hoher Ausbeute und hoher Reinheit erhalten werden kann.

Gegenstand der Erfindung ist demnach ein Verfahren zur Herstellung von (2,5-Dioxo-4-imidazolinyl)harnstoff (Allantoin) der Formel 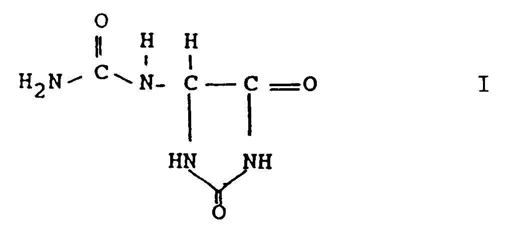
das dadurch gekennzeichnet ist, daß man
a) Glyoxylsäuremethylestermethylhemiacetal der Formel 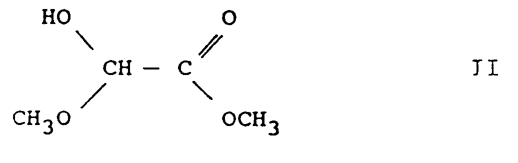 mit Harnstoff in Gegenwart von katalytischen Mengen einer anorganischen Säure bei einem pH-Wert von 1,0 - 2,0 zu einer Verbindung der Formel 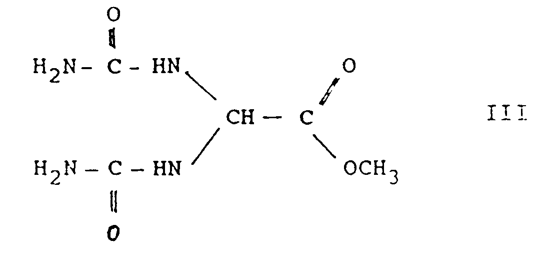 umsetzt, worauf man
b) die Ringschlußreaktion bei einem pH-Wert von 7,0 - 9,0 durchführt.

Das als Ausgangsverbindung verwendete Glyoxylsäuremethylestermethylhemiacetal ist bekannt. Die Verbindung ist leicht zugänglich und kann auf einfache Weise beispielsweise nach EP-B 0 099 981 hergestellt werden.

Die Reaktion wird in einem zweistufigen Verfahren ohne Isolierung des Zwischenprodukts der Formel III durchgeführt. Dabei wird vorerst Glyoxylsäuremethylestermethylhemiacetal mit Harnstoff in Gegenwart von katalytischen Mengen einer anorganischen Säure und gegebenenfalls in Gegenwart eines inerten Verdünnungsmittels umgesetzt. Üblicherweise werden die Ausgangsverbindungen in stöchiometrischer Menge eingesetzt, d. h. pro mol Glyoxylsäuremethylestermethylhemiacetal werden 2 mol Harnstoff eingesetzt. Zur Vervollständigung der Reaktion kann es jedoch vorteilhaft sein, einen bis zu 2 molaren Überschuß an Harnstoff einzusetzen.

Als Kondensationsmsittel werden anorganische Mineralsäuren, beispielsweise Salzsäure oder Schwefelsäure, vorzugsweise Schwefelsäure eingesetzt. Pro mol Glyoxylsäuremethylestermethylhemiacetal werden dabei 0,02 - 0,1 mol, vorzugsweise 0,03 - 0,07 mol Säure eingesetzt.

Gegebenenfalls kann die Reaktion in Gegenwart eines unter Reaktionsbedingungen inerten Verdünnungsmittels durchgeführt werden. Als inerte Verdünnungsmittel kommen niedere aliphatische Alkohole, vorzugsweise Methanol in Betracht. Die Umsetzung erfolgt bei Reaktionstemperaturen von etwa 50 - 80°C.

Nach einer Reaktionszeit von etwa 30 min - 2 Stunden wird gegebenenfalls etwas Verdünnungsmittel abdestilliert, anschließend, das Reaktionsgemisch leicht abgekühlt und zur Durchführung der zweiten Reaktionsstufe mit einer Base, beispielsweise Natronlauge, Kalilauge, Piperidin oder Triethylamin, vorzugsweise mit verdünnter Natronlauge oder Kalilauge bis zu einem pH-Wert von 7,0 - 9,0 versetzt. Anschließend wird das Reaktionsgemisch wieder bis zur Siedetemperatur erwärmt. Gegebenenfalls wird dabei durch weiteres Zudosieren von Lauge der pH-Wert im oben angegebenen Bereich gehalten.

Nach beendeter Reaktion wird das Produkt auf übliche Weise, beispielsweise durch Filtrieren, Zentrifugieren u. dgl. aus der Reaktionslösung isoliert.
Nach dem erfindungsgemäßen Verfahren wird Allantoin in Ausbeuten von bis zu 85 % und in hoher Reinheit erhalten.

### Beispiel 1:

In einem 1200 l-Emailkessel mit Ankerrührer und absteigendem Kühler wurden der Reihe nach unter Rühren 324 kg Glyoxylsäuremethylestermethylhemiacetal, 120 kg Methanol, 540 kg Harnstoff geprillt, 135 kg 8 %ige Schwefelsäure chargiert. Das Gemisch zeigt pH 1,5 - 1,8.

Anschließend wurde der Kessel geschlossen und im Verlaufe von 1 Stunde auf 70°C aufgewärmt. Bei ca. 70°C begann das Gemisch stark zu kochen.

Sobald das Gemisch zu Sieden aufhörte, wurde wieder leicht zugeheizt und insgesamt ca. 60 l Methanol abdestilliert, der Siedepunkt des Gemisches stieg auf ca. 75°C an. Nach etwa 1 Stunde Reaktionszeit bei dieser Temperatur wurde das Gemisch leicht abgekühlt und mit 97 kg 20 %iger NaOH auf pH 7,3 gestellt. Es wurde wieder auf Siedetemperatur erwärmt und durch weitere NaOH-Zugabe der pH-Wert zwischen 7,5 und 8 2 Stunden lang gehalten.

Das Reaktionsgemisch wurde unter Rühren auf mindestens 20°C gekühlt dann zentrifugiert und mit Wasser gewaschen. Das zentrifugenfeuchte Produkt wurde im Vakuum getrocknet.
- Gesamtausbeute:: 359 kg = 84,1 % d. Th.
- Reinheit:: Gehalt: acidimetrisch 99,6 %
Sulfat: 100 ppm
Schmelzpunkt: 220 - 223°C
Sulfatasche : 0,1 %

## Patentansprüche

1. Verfahren zur Herstellung von (2,5-Dioxo-4-imidazolinyl)harnstoff (Allantoin) der Formel 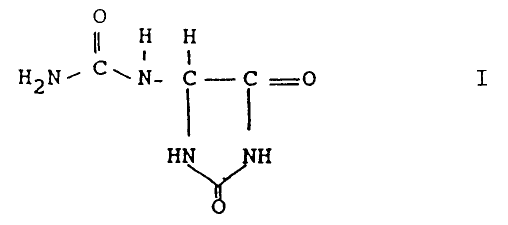 dadurch gekennzeichnet, daß man
a) Glyoxylsauremethylestermethylhemiacetal der Formel 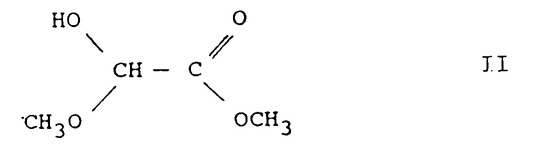 mit Harnstoff in Gegenwart von katalytischen Mengen einer anorganischen Säure bei einem pH-Wert von 1,0 - 2,0 zu einer Verbindung der Formel 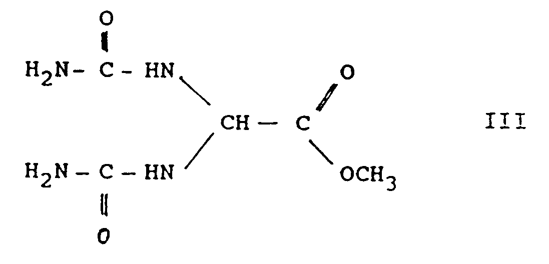 umsetzt, worauf man
b) die Ringschlußreaktion bei einem pH-Wert von 7,0 bis 9,0 durchführt.

2. Verfahren nach Anspruch 1, dadurch gekennzeichnet, daß in Stufe a) pro Mol Glyoxylsäuremethylestermethylhemiacetal 0,02 - 0,1 mol anorganische Säure eingesetzt wird.

3. Verfahren nach einem der Ansprüche 1 oder 2 dadurch gekennzeichnet, daß als anorganische Säure in Stufe a) Schwefelsäure eingesetzt wird.

## Claims

1. Process for the preparation of (2,5-dioxo-4-imidazolinyl)urea (allantoin) of the formula 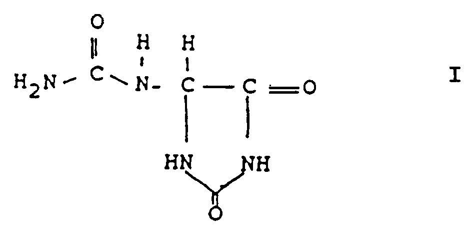 characterized in that
a) methyl glyoxylate methyl hemiacetal of the formula 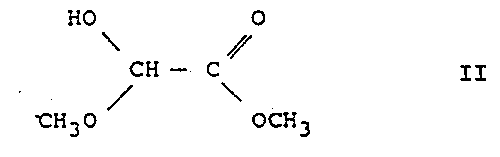 is reacted with urea in the presence of catalytic amounts of an inorganic acid at a pH of 1.0 - 2.0 to give a compound of the formula 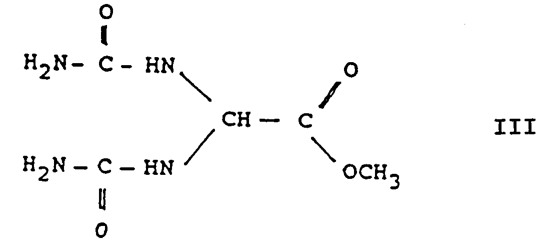 after which
b) the ring-closure reaction is carried out at a pH of 7.0 - 9.0.

2. Process according to Claim 1, characterized in that in stage a) 0.02 - 0.1 mol of inorganic acid is employed per mol of methyl glyoxylate methyl hemiacetal.

3. Process according to Claim 1 or 2, characterized in that sulfuric acid is employed as inorganic acid in stage a).

## Revendications

1. Procédé de fabrication de carbamide (2,5 dioxo-4-glyoxalinyle) (allantoïne) de formule 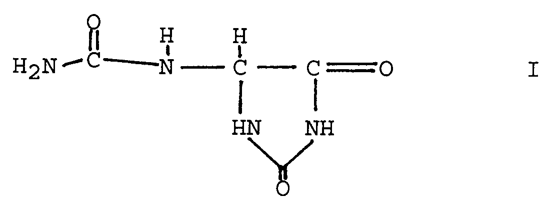 **caractérisé en ce que**
a) on transforme de l'hémiacétal méthylique d'ester méthylique d'acide glyoxylique de formule 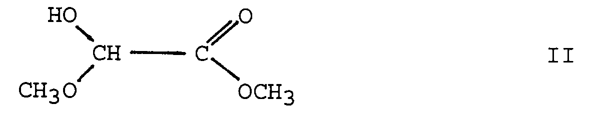 avec du carbamide, en présence de quantités catalytiques d'un acide inorganique, avec une valeur pH de 1,0 - 2,0, en une combinaison de formule 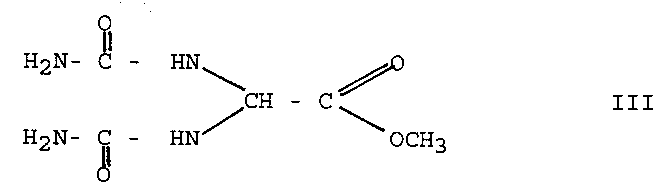 et qu'ensuite
b) on effectue la réaction de cyclisation avec une valeur pH de 7,0 à 9,0.

2. Procédé selon la revendication 1, **caractérisé en ce que,** lors de l'étape a), on utilise par mole d'hémiacétal méthylique d'ester méthylique d'acide glyoxylique, 0,02 - 0,1 mole d'acide inorganique.

3. Procédé selon une des revendications 1 ou 2, **caractérisé en ce qu'**on utilise dans l'étape a), de l'acide sulfurique comme acide inorganique.
